# EUROPÄISCHE PATENTANMELDUNG

(11) **EP 0 585 803 A1**
(43) Veröffentlichungstag der Anmeldung: **09.03.1994**
(21) Anmeldenummer: 93113612.1
(22) Anmeldetag: 26.08.1993
(51) Int. Cl.: A61C 5/06, B65D 25/08, B65D 81/32

(54) **Verfahren zur Herstellung von Mischkapseln zur Zubereitung von Dental-Amalgam**

(30) Priorität: 31.08.1992 CH 2707/92
(71) Anmelder: Ihde, Stefan Klaus Alfred, Dr., CH-8738 Uetliburg (CH)
(72) Erfinder: Ihde, Stefan Klaus Alfred, Dr., CH-8738 Uetliburg (CH)

(57) **Zusammenfassung**

Beschrieben wird ein Herstellungsverfahren für Mehrkomponenten-Mischkapseln aus Glas. Die Herstellung solcher Kapseln erfolgt, ausgehend von einer Flachglasampulle größerer Länge mit einer zirkulären Einschnürung. Wesentliches Erfindungsmerkmal ist die exakte Einhaltung der Befüllungsreihenfolge dahingehend, daß stets zuerst das Flüssigkeitskissen und erst dann das Pulver in die Ampulle gegeben wird. Dadurch gelingt es, die schädliche Strahlungswärme während des Zuschmelzens von wärmeempfindlichen Kissen fernzuhalten. Diese Wärme wird von den Pulverbestandteilen absorbiert oder reflektiert. Das Verfahren eignet sich beispielhaft für die Herstellung von Amalgamkapseln. Weiteres wesentliches Merkmal ist die Instillation von gasförmigem Stickstoff in die Ampulle. Diese Maßnahme verhindert zuverlässig, daß Wasserdämpfe, wie sie in der Atmoshäre von Räumen in denen Gläser mittels Propangas geschmolzen werden in hoher Konzentration üblich sind, in die Kapsel eingeschmolzen werden und somit eine Oxidation der Mischungsbestandteile während der Lagerung des Fertigprodukts erfolgen kann.

## Beschreibung

Die erstmalige Erwähnung von Einmal-Mischkapseln zur vordosierten Darreichung von Dental-Amalgam geht bis in die 1960er Jahre zurück. So erwähnt DE 12 88 243 eine mehrkammerige, teleskopierende Kapsel, die (Zeile 25) auch aus Glas hergestellt werden kann. Dabei kann das Kompartiment, welches das Quecksilber enthält, -meistens ein Kissen aus dünnen Kuststoff-, entweder im Deckel angeordnet sein (DE 12 87 251), andererseits auch völlig lose in der Mischkammer angeordnet sein (DE 28 58 334 C2) oder auch an einer Stelle befestigt sein (DE 33 22 114).

Alle erwähnen Kapseln wurden, obgleich gelegentlich die Möglichkeit der Herstellung aus einem anderen Material, z.B. Glas, erwähnt wurde, stets aus Kunststoff hergestellt.

Auf die Herstellung solcher Kapseln aus Glas geht erstmals die europäische Patentanmeldung 0 476 683 A1 ein. Dort wird eine einkammerige Ampulle mit umlaufender Einschnürung gezeigt, die sowohl die Alloybestandteile als auch ein Kissen, -vorzugsweise aus Kunststoff hergestellt- mit dem Quecksilber enthält.

Es zeigt sich jedoch leider, daß die Herstellung der Mischkapsel gemäss EPA 0 476 683 A1,
mit erheblichen Schwierigkeiten verbunden ist, und daß darüber hinaus, der Ausschuß bei der Herstellung groß ist.

Ferner zeigt sich, daß die beim Zuschmelzen der Ampullen auftretenden Wassergase (aus der Propanverbrennung) die Haltbarkeit einiger Amalgame beeinträchtigen. Dieses Wasser beieinträchtigt einzelne Amalgamlegierungen mehr, andere weniger. Stark beeinträchtigt wird beispielsweise eine Legierung aus 70 % Ag, 18 % Zinn, 12 % Kupfer (Späne 70 %, Kugeln 30).

Vorliegend beschriebenes Herstellungsverfahren minimiert den Ausschuß und sorgt weiterhin dafür, daß die Haltbarkeit der in Glaskapseln eingeschmolzenen Amalgame sich nicht durch das im Rahmen der Herstellung auftretende Wasser beeinträchtigt wird.

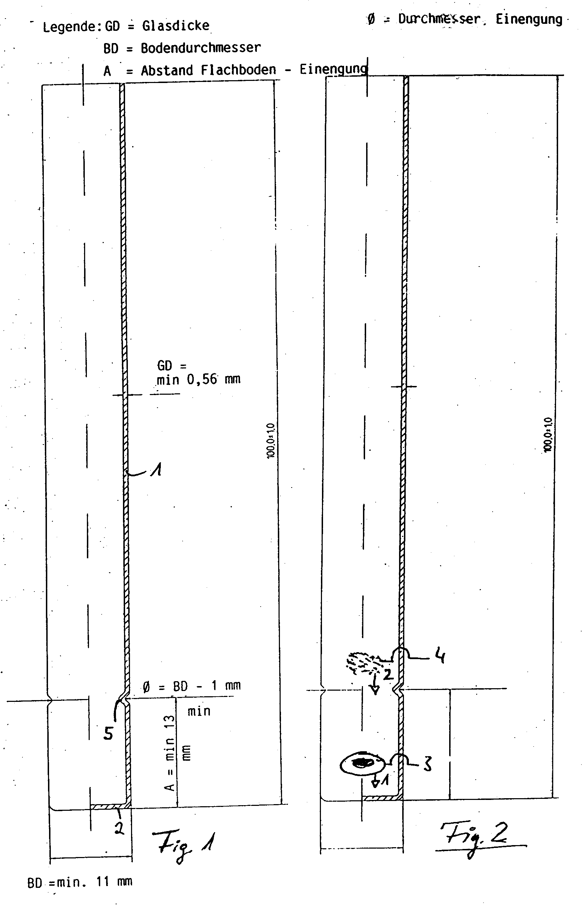

Zunächst wird die prinzipielle Herstellungsweise der Amalgamkapseln aus Glas beschrieben:
Als erster Herstellungsschritt erfolgt die Herstellung eines Flachbodenglases von beispielsweise ca. 10 cm Länge mit einem Bodendurchmesser von beispielsweise 11 mm (Fig. 1). Dieses Flachbodenglas wird als nächstes auf einer Höhe ab Boden von z. B. 13 mm mit einer zirkulären Einschnürung versehen, wobei der Gesamt-Außendurchmesser des Glases sich durch die Einschnürung um mindestens 1 mm verringern soll. Geringer Einschnürungen erschweren das Brechen nach dem Anmischen zum Zwecke der Entnahme.

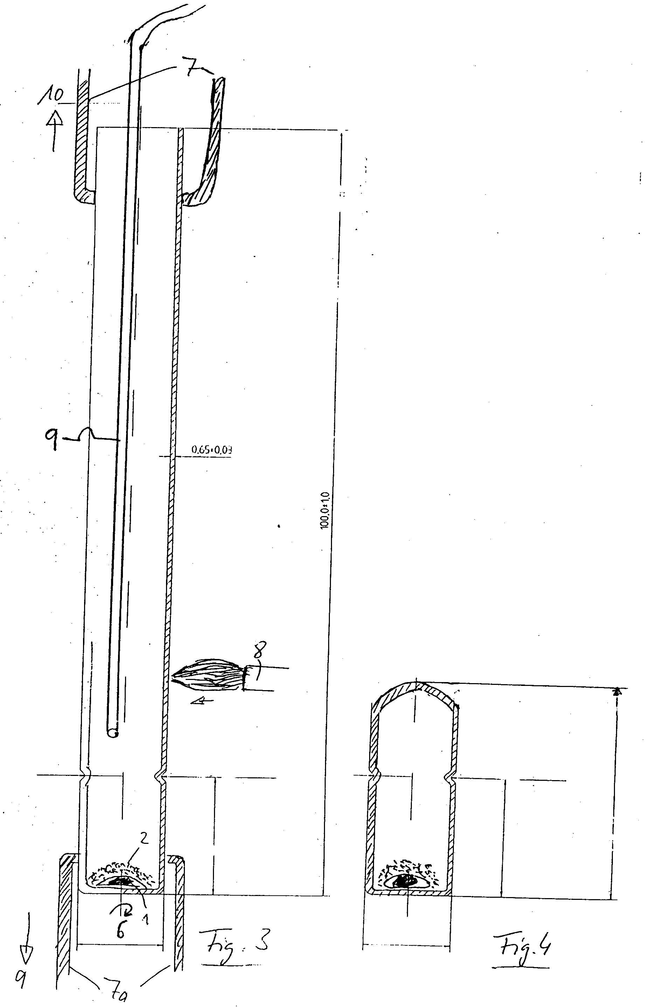

Nun wird das Glas mit Alloy und dem Quecksilberkissen befüllt (Fig. 2), wonach es unter gleichzeitiger und gleichmässiger Drehung und Fixierung am oberen und unteren Ende des Flachbodenglases auf einer Höhe von z. B. weniger als 40 mm (von unten), zugeschmolzen wird (Fig. 3). Die vorteilhafte Höhe der Gesamtkapsel beträgt in diesem Fall rund 31 mm. Diese Höhe gewährleistet, daß die Kapsel in alle herkömmlichen Vibrationsmischer eingebracht werden kann.

Die ersten Herstellungsversuche zeigen jedoch leider, daß durch die Wärme des Zuschmelzvorganges das Quecksilberkissen beieinträchtigt wird. Es zeigen sich folgende Beeinträchtigungen:
1. Deformierungen im Sinne einer Schmelzschrumpfung.
2. Risse im Kissen.
3. Völlige Zerstörung des Kissens mit Austritt von Quecksilber in den Mischraum.

Es bedarf keiner Erwähnung, daß solchermaßen geschädigte Kissen dazu führen, daß die gesamte Kapsel nicht mehr verwendet werden kann. Denn selbst wenn das Kissen nur geschrumpft ist, ohne eigentlich geöffnet worden zu sein, ist nicht sichergestellt, daß die Kapsel den Transport zum Zahnarzt überstehen wird (auf dem Postwege oder durch andere starke Erschüttungerungen kann das geschwächte Kissen platzen), bzw. daß das Kissen während des Mischvorganges geöffnet wird.

Denn die Schrumpfung führt mitunter auch zum umgekehrten Ergebnis:
Durch die Verdickung der Schichtstärke des Kissens kann es dazu kommen, daß die Kräfte der Mischvibration nicht ausreichen, das Kissen während des Mischvorgangs zu zerstören. Damit können die Mischungsbestandteile nicht zueinander gelangen.

Es wurde untersucht, warum das Kissen durch die Zuschmelzwärme geschädigt wird und welche Parameter besonders wichtig sind.

Folgende Ergebnisse wurden erzielt:
1. Ist das Glas dünner als 0,56 mm, gelingt ein Zuschmelzen problemlos auch bei Kapsellängen von 30 mm und darunter. Leider lassen sich diese dünnwandigen Kapseln nicht splitterarm öffnen und diese Kapseln sind daher nicht in der Zahnarztpraxis einsetzbar.
2. Ist die Kapsel länger als 35 mm, spielt der Durchmesser der Glaswandung praktisch keine Rolle mehr. Leider lassen sich solche langen Kapseln nicht mehr in die handelsüblichen Kapselmischer einspannen.

Es zeigt sich jedoch erstaunlicherweise, daß sich Kapseln mit einer Länge von rund 31 mm auch aus brechbarem Glas mit einem Wanddurchmesser von 0,65 mm (+- 0,03 mm) herstellen lassen, wenn bei der Befüllung streng die Reihenfolge derart eingehalten wird,
daß zuerst das Quecksilberkissen in das Flachbodenglas geworfen wird, und danach vorsichtig das Alloypulver auf das Kissen gestreut wird. Nachfolgende Tabelle verdeutlicht das erstaunliche Ergebnis:

| | Versuche | Ausschuß |
|---|---|---|
| Kapselschluss -mit obenliegendem Alloy | 100 | 0 |
| Kapselsschluss -mit obenliegendem Hg-Kissen- | 100 | 15 |
| (Anordnung: 31 mm Kapsellänge, 0,65 mm Wanddurchmesser). | | |

Somit wurde einerseits entdeckt, daß die Wärmeleitung über Glas dem Quecksilberkissen weniger schadet als die Strahlungswärme aus dem Zuschmelzprozess, andererseits, daß sich im Grenzbereich der Kapsellänge um 31 mm durchaus mit indusrieller Genauigkeit Glaskapseln verschließen lassen, deren Wanddurchmesser nur geringfügig über 0,56 mm liegt (nämlich vorliegend um 0,65 mm).

Die solchermaßen hergestellten Kapseln besitzen einen flachen und einen runden Boden (Fig. 4).

In Räumen, in denen Gläser mittels Propangas geschmolzen werden, herrscht eine hohe Luftfeuchtigkeit, was durch die Entwicklung von Wasser im Verbrennungsprozess bedingt ist. Daher erwies es sich als günstig, das oben geschilderte Herstellungsverfahren dahingehend zu modifizieren, daß die Flachbodenampulle vor dem Zuschmelzen, jedoch nicht unbedingt vor dem Befüllen, mit gasförmigem Stickstoff befüllt wird. Dadurch befinden sich die Befüllungspartikel zu jeder Zeit in einer Sauerstoff- und wasserarmen Atmosphäre.

Der Stickstoff hat nicht die Tendenz, sich zu verflüchtigen und daher kann dieses Verfahren auch bei oben geöffneten Ampullen durchgeführt werden.

Die Wirkung dieser Maßnahme verdeutlicht folgende Tabelle:

| Legierung: | |
|---|---|
| 70 % Ag, 18 % Zinn, 12 % Kupfer | Farbveränderung nach 20 Tagen |
| ohne Stickstoff | 30 % |
| mit Stickstoff | 0 % |

Nachfolgend wird unter Bezugnahme auf die Zeichnungen der Herstellungsvorgang erläutert:
- Fig. 1: zeigt die vorteilhaft zu verwendende, rotationssymmetrische Glasampulle (2) (im Schnitt) mit der zirkulären Einschnürung (5), die mind. 1 mm beträgt.
- Fig. 2: zeigt die Befüllung der Ampulle schematisch. Wichtig ist, daß zuerst das Quecksilberkissen (3) eingeworfen wird, und danach vorsichtig das Alloypulver (4) nachgeschüttet wird, wobei das Pulver (4) das Kissen (3) möglichst gleichmässig bedecken soll.
- Fig. 3: zeigt, wie die sich drehende Ampulle zum Zwecke des zuschmelzens gedreht wird (6), dabei festgeahlten durch die Halterungen (7, 7a). Dabei wird die Richtung der Flamme (8) schematisch dargestellt. Diese Fig. zeigt ferner die Instillationspipette (9) für den Stickstoff, die jedoch vor dem eigentlichen Zuschmelzen wieder entfernt werden muß.
- Fig. 4: zeigt die fertige Ampulle im Schnitt. Man erkennt die Befüllung und das kuppelförmige Dach.

## Patentansprüche

1. Verfahren zur Herstellung von Mehrkomponenten-Mischkapseln aus Glas mit den Füllkomponenten Pulver und Flüssigkeit im Kunststoffkissen,
**dadurch gekennzeichnet,**
daß das Flüssigkeitskissen beim Befüllen zuerst in die oben offene Ampulle eingeworfen wird, wonach die Pulverkomponente auf das unten liegende Flüssigkeitskissen gestreut wird.

2. Verfahren nach Anspruch 1,
**dadurch gekennzeichnet,**
daß die Flüssigkeit Quecksilber ist und das Pulver eine zur Herstellung von Dentalamalgam geeignete Legierung darstellt.

3. Verfahren nach Anspruch 1 und 2,
**dadurch gekennzeichnet,**
daß als ein Herstellungsschritt, das Innere des Flachbodenglases mit Stickstoff befüllt wird.
